# EUROPEAN PATENT APPLICATION

(11) **EP 0 787 803 A1**
(43) Date of publication of application: **06.08.1997**
(21) Application number: 96927208.7
(22) Date of filing: 20.08.1996
(51) Int. Cl.: C12P 1/00, C12M 1/40

(54) **IMMOBILIZED ENZYME REACTION METHOD**

(30) Priority: 21.08.1995 JP 211885/95
(71) Applicant: KYOWA HAKKO KOGYO CO., LTD., Chiyoda-ku, Tokyo 100 (JP)
(72) Inventor: TANAKA, Hisao, Yamaguchi 747 (JP); TANAKA, Takayuki, Yamaguchi 747 (JP)
(74) Representative: VOSSIUS & PARTNER
(86) International application number: JP9602327
(87) International publication number: WO9707228

(57) **Abstract**

A method of conducting fixed-bed enzymatic reactions by bringing a substrate into contact with an enzyme immobilized on a carrier packed in a reactor while keeping the optimum temperature of the enzyme, which method comprises conducting the reaction under adiabatic conditions, and simultaneously mixing part of the reaction fluid with a substrate solution and circulating the resultant mixture to the reactor through a heat exchanger, thereby holding the inside of the reactor in a state as homogeneous as possible. The method makes it possible to narrow the temperature range in the reactor and to conduct enzymatic reactions at a temperature near the optimum temperature of the employed enzyme under the condition of a high enzymatic activity.

## Description

### TECHNICAL FIELD

The present invention relates to a method for enzyme reaction, in which a reaction using an enzyme as immobilized on a carrier, that is, a so-called immobilized enzyme, is carried out under adiabatic condition while a part of the resulting reaction mixture is re-circulated into the reactor through a heat exchanger to thereby ensure the high enzyme activity for a long period of time and maintain the high productivity with said enzyme.

### BACKGROUND ART

It is known that an immobilized enzyme may be used for enzyme reaction, and it is also known that such an immobilized enzyme is preferably reacted with a substrate at a temperature most preferred for the enzyme in order to ensure the high enzyme activity.

The reaction of an enzyme with a substrate is exothermic or endothermic, and the enzyme reaction system must be heated or the heat resulting from the enzyme reaction must be removed in order to maintain high enzyme activity. To heat such a enzyme reaction system or to remove the heat resulting from such enzyme reaction, for example, generally employed is a method of introducing constant-temp. water into jacket as provided around a reactor. In this method, since the heat transferring rate in the direction vertical to the flow of the reaction mixture is small, and the temperature distribution in said vertical direction is uneven. In other words, in this method for exothermic reaction, the temperature of the area around the wall in the reactor is low while the temperature of center area in the reactor is high. For a large-sized reactor for industrial use, even if it is cooled during exothermic reaction through such a jacket system, the temperature of the center area in the reactor will increase greatly.

A device is known which is equipped with a cooling duct, etc. as inserted into a reactor (J. Ferment. Technol., 63, 371, 1985). However, the device of that type is problematic in that its inner structure is complicated, in that the cost for its installation is high, and in that the exchange of the immobilized enzyme with a new one requires troublesome operations. Therefore, the device is not suitable for industrial use.

Under the situation, it is desired to develop an industrial method for immobilized-phase reaction using an enzyme-immobilized carrier, in which the temperature in the reactor is kept as even as possible throughout the reactor thereby realizing the temperature most suitable for the enzyme in order to ensure high enzyme activity.

### DISCLOSURE OF THE INVENTION

It has been studied various means of enzyme reaction in order to attain the reduction in the difference in temperature between the center area in the reactor and the area around the wall in the reactor and the reduction in the difference in temperature between the area around the substrate inlet positioned at the top of a reactor and the area around the outlet positioned at the bottom thereof. As a result, it has been found that the former may be attained by conducting the enzyme reaction under adiabatic condition, while the latter may be attained by circulating the reaction mixture through the reaction system.

According to the present invention, for enzyme reaction through contact of a substrate with an enzyme as immobilized onto a carrier filled in a reactor, said reaction is conducted under adiabatic condition while a part of the reaction mixture is mixed with a substrate solution and re-circulated into the reaction phase through a heat exchanger, whereby the temperature in the reactor is kept to be a suitable one for the enzyme reaction to ensure the high enzyme activity and increase reaction efficiency.

The adiabatic reaction may result in unifying the temperature in the direction vertical to the flow of the reaction mixture; while the re-circulation of the reaction mixture into the reactor may result in increasing the flow of the reaction mixture in the reactor, thereby increasing the heat capacity of the reaction mixture to reduce the temperature difference between the area around the inlet of the reactor and that around the outlet of the reactor.

The reactor used in the present invention may be of any type that is covered with a thermal insulation material such as glass wool, and can block the heat transfer between the inside of the reactor and the outside thereof. The heat exchanger used in the present invention may be of any type that has the function to heat or cool the substrate solution.

A substrate solution may be put into the reactor via one or more inlets. If desired, a plurality of heat exchangers may. be fixed to the apparatus, and a plurality of substrate solutions as prepared and conditioned at different temperatures may be separately put into the reactor through such plural heat exchangers. In that manner, the temperature of the reaction phase may be unified more.

The method of the present invention can apply to any and every enzyme reaction. In the reaction, any and every enzyme as immobilized by any per-se known method is used, and advantageously used herein are enzymes as immobilized onto carrier particles.

For example, the present invention may apply to a method of producing aspartic acid, which comprises immobilizing aspartase onto an ion-exchange resin, and put a solution of ammonium fumarate into a reactor filled with said immobilized enzyme to conduct the enzyme reaction between the enzyme and the substrate. The details of this method are concretely illustrated in Example shown below.

### BRIEF DESCRIPTION OF THE DRAWINGS

Fig. 1 is a graphical view showing one embodiment of the pipe line of a reactor used in carrying out the present invention.

In the figure; 1 is a heat exchanger; 2 is a reactor; 3, 4, 7 and 8 are thermometric resistors; 5 and 9 are pumps; 6, 11 and 13 are flow meters; and 10 and 12 are valves.

Fig. 2 is an outline view showing the adiabatic reactor as used in Example.

Fig. 3 is a graph showing the temperature changes in Example in the area around the inlet of the reactor, in the area around the outlet of the reactor, in the center area in the reactor and in the area around the wall of the reactor, after the substrate is put into the reactor.

Fig. 4 is a graph showing the temperature changes in Refernce Example 1 in the area around the inlet of the reactor, in the area around the outlet of the reactor, in the center area in the reactor and in the area around the wall of the reactor, after the start is putinto the reactor.

Fig. 5 is an outline view showing the jacket-combined reactor as used in Reference Example 2.

Fig. 6 is a graph showing the temperature changes in Reference Example 2 in the area around the inlet of the reactor, in the area around the outlet of the reactor, in the center area in the reactor and in the area around the wall of the reactor, after the substrate is put into the reactor.

Fig. 7 is a graph showing the influence of the temperature on its enzymatic activity.

### BEST MODES OF CARRYING OUT THE INVENTION

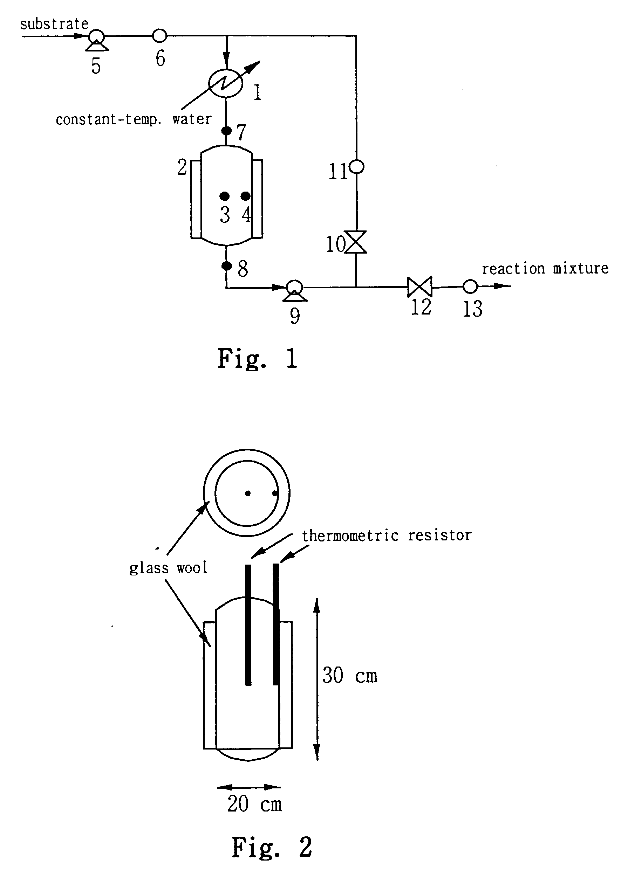

The present invention is described in detail with reference to Fig. 1 attached hereto.

In Fig. 1, a substrate is heated or cooled in the heat exchanger 1 via the pump 5 and the flow meter 6, and then put into the reactor 2 which is covered with a thermal insulation material. The reaction mixture is discharged out from the lower part of the reaction phase, and a part of the discharged reaction mixture is circulated into the heat exchanger via the pump 9 and then via the valve 10 and the flow meter 11. The thermometric resistors 7, 8, 3 and 4 as fixed around the inlet, the outlet, the center part and the wall of the reactor measure the temperature of each area.

One embodiment of carrying out the invention using the device is described in detail below.

### EXAMPLE

According to the method described in Journal of Solid-Phase Biochemistry, 3, 247 (1978), aspartase was extracted from the cultivated cells of microorganism producing aspartase, and introduced into a column filled with an ion-exchange resin, Duolite A-7 (produced by Diamond Shamrock Co.), whereby the enzyme was immobilized onto the resin.

The obtained enzyme-immobilized carrier was filled in the adiabatic reactor of Fig. 2. Two thermometric resistors were fixed inside the reactor both at the depth of 16 cm from the top of the reaction phase, while one was in the center of the reactor and the other was adjacent to the wall of the reactor.

A substrate, 1 M ammonium fumarate solution as adjusted to pH 8.5 with aqueous ammonia, was put into the reactor at a flow rate of 2 liter/hour, while the reaction mixture was re-circulated into the reactor at a flow rate of 2 liter/hour.

Constant-temp. water at 25°C was passed through the heat exchanger. The concentration of fumaric acid in the reaction mixture in a steady state was measured at regular intervals. The conversion rate of fumaric acid into aspartic acid was almost 100 %.

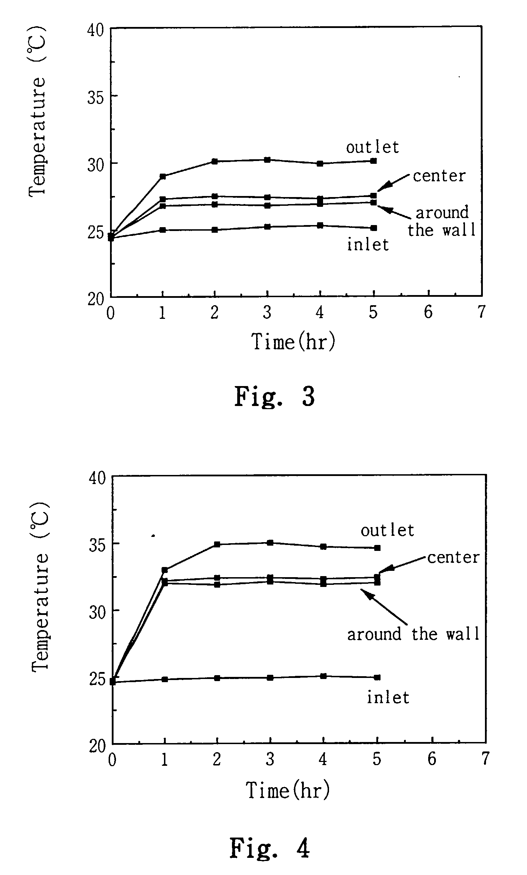

The temperature changes in the area around the inlet of the reactor, in the area around the outlet of the reactor, in the center area in the reactor and in the area around the wall of the reactor are shown in Fig. 3.

The difference between the temperature in the area around the inlet of the reactor and that in the area around the outlet of the reactor was 4.7°C; while that between the temperature in the center area in the reactor and that in the area around the wall of the reactor was 0.5°C.

The temperature difference in the direction vertical to the flow of the reaction mixture, that is, the difference between the temperature in the center area in the reactor and that in the area around the wall of the reactor is extremely small; while the temperature difference in the direction of the flow of the reaction mixture, that is, the difference between the temperature in the area around the inlet of the reactor and that in the area around the outlet of the reactor was also small. Thus, it was possible to keep the temperature in the reactor within the most suitable range for the enzyme reaction.

As shown in Reference Example 3, the immobilized enzyme could more hardly retain its enzyme activity to attain high conversion with the increase in the temperature around it. Therefore, it is important to keep the temperature in the reactor as even as possible within the most suitable range for the enzyme reaction.

It has now been verified that the method demonstrated above is effective for unifying the temperature in the reactor as much as possible within the most suitable range for the enzyme employed, by suitably controlling the flow rate of the substrate solution in the reactor and the flow rate of the reaction mixture re-circulated into the reactor in accordance with the type of the reactor used.

In addition, according to the method demonstrated above, it is possible to obtain the reaction product at any desired concentration by suitably controlling the flow rate of the substrate solution in the reactor and the flow rate of the reaction mixture re-circulated into the reactor.

### REFERENCE EXAMPLE 1

The same process as in the above-mentioned Example was repeated, except that the valve 11 was completely shut to stop the re-circulation of the reaction mixture into the reactor.

The temperature changes in the area around the inlet of the reactor, in the area around the outlet of the reactor, in the center area in the reactor and in the area around the wall of the reactor are shown in Fig. 4.

The difference between the temperature in the center area in the reactor and that in the area around the wall of the reactor was kept small; but the difference between the temperature in the area around the inlet of the reactor and that in the area around the outlet of the reactor was about 9.8°C. Thus, in this system, it was impossible to unify the temperature in the reactor within the most suitable range for the enzyme reaction.

The difference between the temperature in the area around the inlet of the reactor and that in the area around the outlet of the reactor may be reduced by increasing the flow rate of the substrate solution, however, the time for the contact of the substrate with the enzyme shall be shortened, resulting in insufficient enzyme reaction.

If the concentration of the substrate is lowered in order to attain sufficient enzyme reaction, the concentration of the intended product obtained is lowered, and some complicated operations are required for collecting the final product.

### REFERENCE EXAMPLE 2

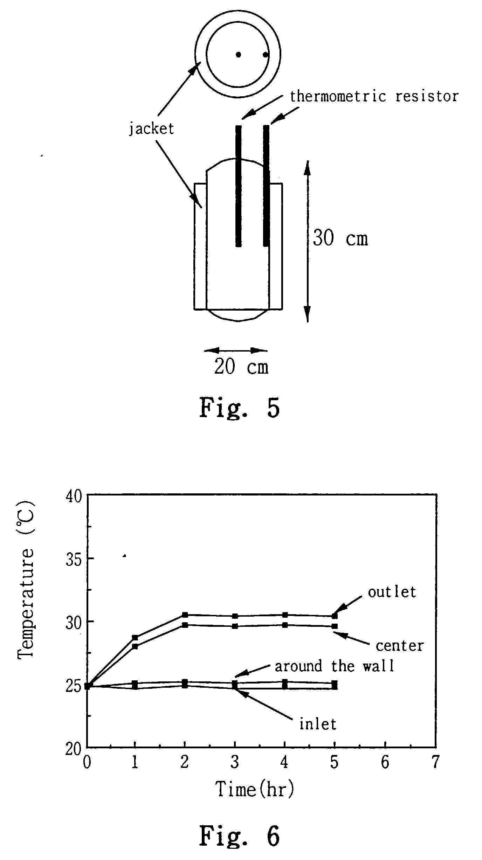

Five liter of immobilized aspartase prepared in the same manner as in the above-mentioned Example was filled into a water-cooling jacket-combined reactor of Fig. 5, which has the same appearance as that of the reactor employed in Example; and the same substrate as in Example was put into the reactor under the same conditions as those in Example, except that the reaction mixture was not re-circulated into the reactor.

Constant-temp. water at 25°C was passed through the jacket at a constant flow rate in order to cool the reactor.

The temperature changes in the area around the inlet of the reactor, in the area around the outlet of the reactor, in the center area in the reactor and in the area around the wall of the reactor are shown in Fig. 6.

The temperature difference in the direction of the flow of the reaction mixture was about 4.8°C, which is almost on the same level as in the above-mentioned Example. However, the temperature difference in the direction vertical to the flow of the reaction mixture was about 5.3°C, which is much larger than that in said Example.

The temperature difference in the direction vertical to the flow of the reaction mixture will be much larger in industrial-scale reactors. Thus, it is understood that the reaction system employed in Reference Example 2 is not suitable as the reactor for immobilized enzymes.

### REFERENCE EXAMPLE 3

The influence of the temperature on enzymatic activity of an immobilized enzyme was evaluated herein.

Immobilized aspartase prepared in the same manner as in the above-mentioned Example was filled into a column (1 cm x 15 cm) and kept at 17°C, 27°C or 37°C, into which was introduced a 200 g/liter ammonia fumarate solution, at a flow rate of 0.05 ml/min. The aspartic acid content in the reaction mixture in the column was measured at regular intervals, from which was obtained the conversion rate.

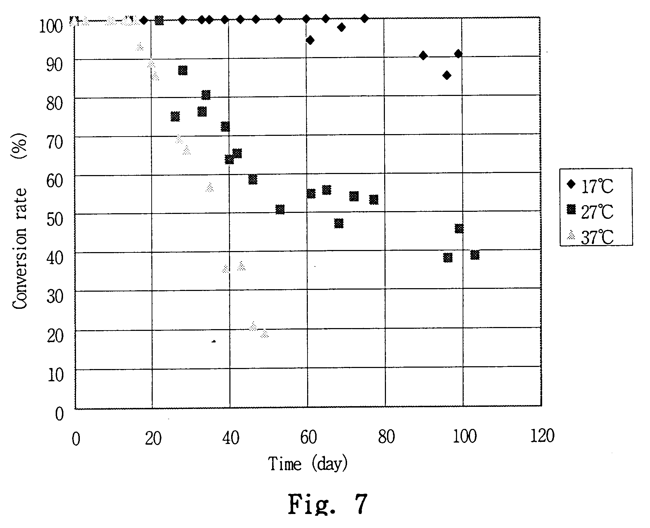

The data obtained are shown in Fig. 7.

Fig. 7 shows that the enzyme activity of the immobilized enzyme is greatly lowered with the increase in the temperature of column, resulting in the decrease in the conversion rate.

### INDUSTRIAL APPLICABILITY

According to the method of the present invention for enzyme reaction, the temperature difference in the reactor can be reduced and, the enzyme reaction can be conducted around the temperature most suitable for the enzyme, whereby the enzyme reaction is conducted efficiently, and the productivity is improved.

## Claims

1. A method for enzyme reaction conducted through contact of an enzyme as immobilized onto a carrier filled in a reactor with a substrate, which is characterized in that said enzyme reaction is conducted under adiabatic condition while a part of the reaction mixture is mixed with a substrate solution and re-circulated into the reactor through a heat exchanger.
